(19) Europäisches Patentamt
European Patent Office
Office européen des brevets

(11) Publication number: **0 058 505**
**B1**

(12) **EUROPEAN PATENT SPECIFICATION**

(45) Date of publication of patent specification: **29.05.85**

(21) Application number: **82300625.9**

(22) Date of filing: **09.02.82**

(51) Int. Cl.$^4$: **C 07 C 120/00,**
C 07 C 121/75,
C 07 D 213/64, B 01 J 31/02 //
A01N53/00

# (54) Preparation of esters containing an alpha-cyano group in the alcohol portion of the esters.

(30) Priority: **12.02.81 US 233756**
**12.02.81 US 233808**
**12.02.81 US 233795**

(43) Date of publication of application:
**25.08.82 Bulletin 82/34**

(45) Publication of the grant of the patent:
**29.05.85 Bulletin 85/22**

(84) Designated Contracting States:
**CH DE FR GB IT LI NL**

(56) References cited:
**EP-A-0 026 542**
**DE-A-2 825 197**
**US-A-4 110 361**
**US-A-4 110 362**
**US-A-4 110 663**
**US-A-4 280 965**

(73) Proprietor: **FMC Corporation**
**2000 Market Street**
**Philadelphia Pennsylvania 19103 (US)**

(72) Inventor: **Baum, Jonathan Sheffield**
**148 South Main Street**
**Pennington New Jersey 08534 (US)**

(74) Representative: **Crampton, Keith John Allen et al**
**D YOUNG & CO 10 Staple Inn**
**London WC1V 7RD (GB)**

**EP 0 058 505 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing esters of carboxylic acids, more specifically, esters which contain a cyano group bonded to the alpha-carbon atom in the alcohol portion of the ester molecule.

Esters with a cyano group so situated are prepared by reacting an acid with the appropriate cyanohydrin. According to U.S. 3,835,176, the reaction can also be effected by treating an acyl halide with a mixture of the appropriate aldehyde and aqueous sodium or potassium cyanide, optionally in an aprotic solvent. It is disclosed, for example, that 3-phenoxy-α-cyanobenzyl chrysanthemate is prepared in 64% yield by reacting chrysanthemoyl chloride, 3-phenoxybenzaldehyde, and an aqueous solution of sodium cyanide at 0°C for 1 hour.

U.S. 4,110,361 discloses a first variation of this process that uses, in addition to the acyl halide, the aldehyde, and the water-soluble cyanide, a mixture of water, a water-immiscible aprotic solvent and a surface-active agent as catalyst. Using a preferred non-ionic poly(ethylenoxy) compound as the catalyst, for example, the commercial insecticidal ester, α-cyano-3-phenoxybenzyl 3-(2,2-dichloroethenyl)-2,2-dimethylcyclopropanecarboxylate, is prepared in 80% yield at room temperature in a reaction time of two hours.

U.S. 4,110,362 discloses a second variation of this process that uses, in addition to the acyl halide, the aldehyde, and the water-soluble cyanide, a mixture of water, a water-immiscible aprotic solvent, an "onion" e.g., a quaternary ammonium, catalyst. This variation shortens the reaction time and increases the yield sufficiently to make the process a candidate for the commercial production of insecticidal esters. However, the onium catalysts are relatively expensive.

U.S. 4,110,363 discloses a third variation of this process that uses, in addition to the acyl halide, the aldehyde, and the water-soluble cyanide, a mixture of water, a water-immiscible aprotic solvent, and a macrocyclic polyether or "crown ether" catalyst. Using the preferred 18-crown-6 as the catalyst, for example, the commecial insecticidal ester, α-cyano-3-phenoxybenzyl 3-(2,2-dichloroethenyl)-2,2-dimethylcyclopropanecarboxylate, is prepared in 76% yield at room temperature in a reaction time of two hours.

European Application EP—A1—26 542 describes processes for the production of alpha - cyano - 3 - phenoxybenzyl 3 - (2,2 - dichloroethenyl) - (2,2 - dimethylcyclopropanecarboxylate, alpha - cyano - 3 - phenoxybenzyl 3 - (2,2 - dibromoethenyl) - 2,2 - dimethylcyclopropanecarboxylate, alpha - cyano - 3 - phenoxybenzyl 3 - (2 - chloro - 3,3,3 - trifluoropropenyl) - 2,2 - dimethyl - cyclopropanecarboxylate, alpha - cyano - 3 - phenoxybenzyl 2,2,3,3 - tetramethylcyclopropanecarboxylate, and alpha - cyano - 3 - phenoxybenzyl 2 - (4 - chlorophenyl) - 3 - methylbutanoate by reacting an acyl halide with an aldehyde in a mixture of substantially water-immiscible aprotic solvent and an aqueous solution of water-soluble cyanide salt in the presence of a catalytic amount of rate-promoting agent that is a tertiary amine or polyamine, cryptate, amphoteric surfactant, or acid salt of a tertiary amine.

German Specification DE—A1—28 25 197 discloses a number of alternative processes for preparing insecticidal alpha-cyano esters.

Were it possible to shorten the reaction time and increase the yield, producing this and other alpha-cyano esters by reacting an acyl halide with an aldehyde and a cyanide would be of great commercial interest. Insecticidal alpha-cyano esters whose preparations would be facilitated include α - cyano - 3 - phenoxybenzyl 3 - (2,2 - dichloroethenyl) - 2,2 - dimethylcyclopropanecarboxylate and α - cyano - 3 - phenoxybenzyl 3 - (2,2 - dibromoethenyl) - 2,2 - dimethylcyclopropanecarboxylate. Other insecticidal alpha-cyano esters of particular interest are α - cyano - 3 - phenoxybenzyl 3 - (2 - chloro - 3,3,3 - trifluoropropenyl) - 2,2 - dimethylcyclopropanecarboxylate, α - cyano - 3 - phenoxybenzyl 2,2,3,3 - tetramethylcyclopropanecarboxylate, α - cyano - 3 - phenoxybenzyl 2 - (4 - chlorophenyl) - 3 - methylbutanoate, cyano - (3 - phenoxyphenyl)methyl 4 - difluoro - methoxy - α - 1 - methylethyl)benzeneacetate, cyano - (4 - fluoro - 3 - phenoxyphenyl)methyl 3 - (2,2 - dichloroethenyl) - 2,2 - dimethylcyclopropanecarboxylate, cyano - (6 - phenoxypyrid - 2 - yl)methyl 3 - (2,2 - dichloroethenyl) - 2,2 - dimethylcyclopropanecarboxylate, and cyano - (3 - phenoxyphenyl)methyl 2 - [2 - chloro - 4 - trifluoromethylphenyl)amino] - 3 - methylbutanoate, as well as cyano - [3 - (4 - halophenoxy)phenyl] - methyl 3 - (2,2 - dichloroethenyl) - 2,2 - dimethylcyclopropanecarboxylates, where halo is fluorine, chlorine or bromine.

One advantage of this invention is that it provides a process for making alpha-cyano esters in very high yield in a short time. Another advantage of this invention is that it provides an esterification process whose product does not require lengthy and expensive purification.

Accordingly, this invention provides a method of preparing an alpha-cyano ester other than alpha - cyano - 3 - phenoxybenzyl 3 - (2,2 - dichloroethenyl) - 2,2 - dimethylcyclopropanecarboxylate, alpha - cyano - 3 - phenoxybenzyl 3 - (2,2 - dibromoethenyl) - 2,2 - dimethylcyclopropane - carboxylate, alpha - cyano - 3 - phenoxybenzyl 3 - (2 - chloro - 3,3,3 - trifluoropropenyl) - 2,2 - dimethylcyclopropanecarboxylate, alpha - cyano - 3 - phenoxybenzyl 2,2,3,3 - tetramethylcyclopropanecarboxylate, and alpha - cyano - 3 - phenoxybenzyl 2 - (4 - chlorophenyl) - 3 - methylbutanoate, comprising reacting an acyl halide with an aldehyde in a mixture of substantially water-immiscible aprotic solvent and an aqueous solution of water-soluble cyanide salt in the presence of a catalytic amount of rate-promoting agent that is a tertiary amine or polyamine, a cryptate, an amphoteric aminoalkylsulfonic acid surfactant, or

acid salt of a tertiary amine. Either the acyl halide or the aldehyde may exhibit optical or geometric isomerism, which is not affected by the reaction.

In a preferred embodiment of the present invention, an insecticidal α-cyano-3-phenoxybenzyl ester of the formula

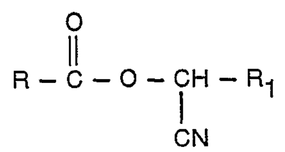

in which either (a) R is 3 - (2,2 - dichloroethenyl) - 2,2 - dimethylcyclopropyl, 3 - (2,2 - dibromoethenyl) - 2,2 - dimethylcyclopropyl, 3 - (2 - chloro - 3,3,3 - trifluoropropenyl) - 2,2 - dimethylcyclopropyl, 2,2,3,3 - tetramethylcyclopropyl, 1 - (4 - chorophenyl) - 2 - methylpropyl, 1 - (4 - difluoromethoxy - phenyl) - 2 - methylpropyl, or 1 - [(2 - chloro - 4 - trifluoromethylphenyl) - amino] - 2 - methylpropyl and $R_1$ is 4 - fluoro - (3 - phenoxy)phenyl, 3 - (4 - halophenoxy)phenyl or 6 - phenoxy - 2 - pyridyl; or (b) R is 1 - (4 - difluoromethoxy - phenyl) - 2 - methylpropyl or 1 - [(2 - chloro - 4 - trifluoromethyl)amino] - 2 - methylpropyl and $R_1$ is 3-phenoxyphenyl, is prepared by reacting an acyl halide of the formula R—CO—X, with an aldehyde of the formula $R_1$—CH=O, where R and $R_1$ are as defined above.

The process of this invention is especially effective in producing a high yield of insecticidal α-cyano-3-phenoxybenzyl esters in a short time when the rate-promoting agent is a tertiary polyamine or cryptate and R is 3 - (2,2 - dichloroethenyl) - 2,2 - dimethylcyclopropyl, 3 - (2,2 - dibromoethenyl) - 2,2 - dimethylcyclopropyl, or 3 - (2 - chloro - 3,3,3 - trifluoropropenyl) - 2,2 - dimethylcyclopropyl, or when the rate-promoting agent is an amphoteric aminoalkyl sulfonic acid surfactant and R is 3 - (2,2 - dichloroethenyl) - 2,2 - dimethylcyclopropyl or 1 - (4 - chlorophenyl) - 2 - methylpropyl, or when the rate-promoting agent is an acid salt of a tertiary amine and R is 3 - (2,2 - dichloroethenyl) - 2,2 - dimethylcyclopropyl, 3 - (2,2 - dibromoethenyl) - 2,2 - dimethylcyclopropyl, or 3 - (2 - chloro - 3,3,3 - trifluoropropenyl) - 2,2 - dimethylcyclopropyl. Outstanding results are obtained when R is 3 - (2,2 - dichloroethenyl) - 2,2 - dimethylcyclopropyl.

Although the process of this invention is especially advantageous when R is selected from the groups named above, the process is also effective in producing other alpha-cyano esters in which R is an aliphatic or aromatic group, which may optionally contain various substituents. Although the process of this invention is preferably used to produce α-cyano-3-phenoxybenzyl esters by using 3-phenoxybenzaldehyde as a reactant, the process is equally suited to the production of other alpha-cyano esters by varying the type of aldehyde employed in the process.

Various aprotic solvents which are substantially water-immiscible may be used in the process. Any alkyl, haloalkyl, aryl, haloaryl, aralkyl, haloaralkyl, or cyclic hydrocarbon, provided that it is a liquid at temperatures between about 0°C and 50°C and forms a discrete second phase when mixed with water, may be used. Such solvents include iso-hexane, 3-methylpentane, 2,3-dimethylbutane, 2,2-dimethylbutane, n-heptane, n-octane, petroleum ether, ligroin, n-propyl bromide, n-propyl iodide, n-butyl chloride, n-butyl bromide, n-pentyl chloride, n-pentyl bromide, diethyl ether, dipropyl ether, dibutyl ether, benzene, toluene, and xylene, for example. Among these solvents, n-heptane is preferred because it is readily available and inexpensive.

A number of water-soluble cyanide salts may be used; for example, the salt may be an alkali metal cyanide such as lithium, sodium, potassium, rubidium, or cesium cyanide, or mixtures thereof. Among these, sodium cyanide generally is preferred. However, when it is desired to use certain cryptates as rate-promoting agents, it may be desirable to substitute lithium or potassium cyanide as described below.

The cyanide salt is dissolved in water, the amount of water employed being relatively small, but preferably sufficient to keep all of the cyanide salt in solution under the reaction conditions. In the case that the salt is sodium cyanide, the preferred molar ratio of water to sodium cyanide is between about 3.5 and 6, preferably about 4.5.

The process of this invention is conducted in the presence of a catalytic amount of rate-promoting agent selected from tertiary amines, polyamines, cryptates, amphoteric surfactants, and acid salts of tertiary amines. For purposes of this invention, a catalytic amount of rate-promoting agent is in the range 1—5 mole percent based on aldehyde, advantageously about 2 mole percent.

The rate-promoting agent may be a tertiary amine of polyamine, a tertiary polyamine being a compound containing more than one tertiary amino nitrogen atom. Particularly desirable tertiary polyamines are linear tertiary polyamines of the formula

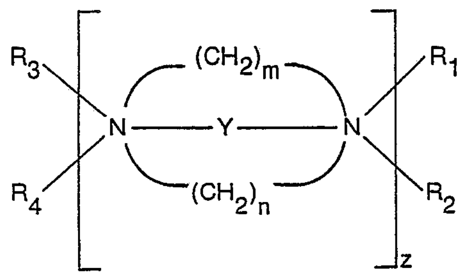

wherein Y is $-(CH_2)_k-$ with k being 1—6, $C_3$—$C_7$ cycloalkane, $C_2$—$C_6$ alkenyl, or $C_2$—$C_6$ alkynyl; z is 1 or 2, and when z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R_4$ are absent.

Particularly useful linear tertiary polyamines within the aforesaid description are 2,4-dimethyl-2,4-diazapentane, 2,5-dimethyl-2,5-diazahexane, 1,1′-(1,2-ethanediyl)bis[piperidine], N,N,N′,N′-tetramethyl-1,2-diaminocyclohexane, 1,4-dimethyl-1,4-diazacyclohexane, diazabicyclo[2.2.2]octane, 2,6-dimethyl-2,6-diazaheptane, 2,7-dimethyl-2,7-diazaoctane, 2,7-dimethyl-2,7-diaza-4-octene, 2,7-dimethyl-2,7-diaza-4-octyne, 2,9-dimethyl-2,9-diazadecane, and 2,5,8,11-tetramethyl-2,5,8, 11-tetraazadodecane. Among these compounds, diazabicyclo[2.2.2]octane, 2,6-dimethyl-2,6-diazaheptane, 2,7-dimethyl-2,7-diazaoctane, and 2,5,8,11-tetramethyl-2,5,8,11-tetrazadecane are preferred, and diazabicyclo[2.2.2]octane is especially attractive.

Macrocyclic tertiary polyamines such as 1,4,8,11-tetraazacyclotetradecane, for example, are also useful, as are sparteine and hexamethylenetetraamine.

Cryptates, polyoxadiazamacrobicycles, constitute another class of rate-promoting agent useful in the practice of this invention. Cryptates of the formula

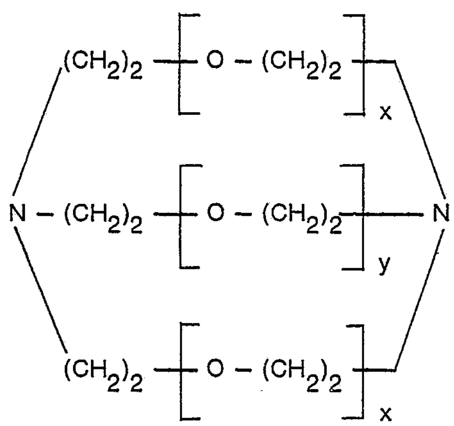

wherein x and y are independently 1 or 2 are especially useful. When the cyanide salt is sodium cyanide 4,7,13,16,21-pentaoxa-1,10-diazabicyclo[8.8.5]tricosane is preferred, but when lithium cyanide is employed 4,7,13,18-tetraoxa-1,10-diazabicyclo[8.5.5]eicosane should be used, and 4,7,13,16,21,24-hexaoxy-1,10-diazacyclo[8.8.8]hexacosane is preferred when potassium cyanide is employed.

Further, for purposes of this invention and wherever it appears in the specification or claims, the term "aminoalkylsulfonic acids", means compounds having the structural formula

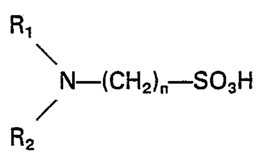

wherein n is 2 or 3, and wherein $R_1$ and $R_2$ are substituents selected collectively to render the aminoalkylsulfonic acids soluble in water-immiscible aprotic solvents. Suitable substituent groups, which collectively contain hydrocarbon units, are present in the following specific aminoalkylsulfonic acids: 1,4-piperazinebisethanesulfonic acid, 4-pyridineethanesulfonic acid, 2 - [N,N - di - (2 - hydroxy)ethyl]aminoethanesulfonic acid, 3 - (cyclohexylamino)propanesulfonic acid, 2 - [4 - (2 - hydroxyethyl)piperizine - 1 - yl)]-ethanesulfonic acid, 2 - (N - morpholino)ethanesulfonic acid, N - tris(hydroxymethyl)methyl - 2 - aminoethanesulfonic acid, 2 - (N - morpholino)propanesulfonic acid, and N - tris(hydroxymethyl)methyl - 2 - aminoethanesulfonic acid. Among these compounds 1,4-piperizinebisethanesulfonic acid and 3-(cyclohexylamino)propanesulfonic acid are preferred, and 3-(cyclohexylamino)propanesulfonic acid is especially effective.

The tertiary amines whose acid salts are rate-promoting agents contain one or more, e.g., two, nitrogen atoms. For purposes of this invention and wherever it appears in the specification or claims the term, "acid salts of tertiary amines", means products of the reaction between a strong acid, such as HCl, HBr, $H_2SO_4$, $HBF_4$, $HClO_4$, or HCN and a tertiary amine or polyamine, a tertiary polyamine being a compound containing more than one tertiary amino nitrogen atom.

For purposes of this invention, a tertiary amine has the structural formula

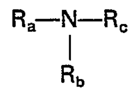

wherein $R_a$, $R_b$, and $R_c$ are hydrocarbon groups.

Particularly desirable tertiary polyamines within the scope of this invention are linear tertiary polyamines of the formula

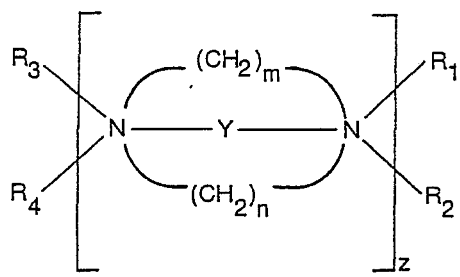

wherein Y is $—CH_2)_k—$ with k being 1—6, $C_3—C_7$ cycloalkane, $C_2—C_6$ alkenyl, $C_2—C_6$ alkynyl; z is 1 or 2, and when z is 1, m and n are 0 or independently 1—6, and when m and n are 0, $R_1$, $R_2$, $R_3$ and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R^4$ are absent.

Particularly useful linear tertiary polyamines within the aforesaid description are 2,4-dimethyl-2,4-diazapentane, 2,5-dimethyl-2,5-diazahexane, 1,1′-(1,2-ethanediyl)bis[piperidine], N,N,N′,N′-tetramethyl-1,2-diaminocyclohexane, 1,4-dimethyl-1,4-diazacyclohexane, diazabicyclo[2.2.2]octane, 2,6-dimethyl-2,6-diazaheptane, 2,7-dimethyl-2,7-diazaoctane, 2,7-dimethyl-2,7-diaza-4-octene, 2,7-dimethyl-2,7-diaza-4-octyne, 2,9-dimethyl-2,9-diazadecane, and 2,5,8,11-tetramethyl-2,5,8,11-tetraazadodecane. Among these compounds diazabicyclo[2.2.2]octano, 2,6-dimethyl-2,6-diazaheptane, 2,7-dimethyl-2,7-diazaoctane, and 2,5,8,11-tetramethyl-2,5,8,11-tetrazadecane are preferred, and diazabicyclo[2.2.2]octane is especially attractive.

Macrocyclic tertiary polyamines such as 1,4,8,11-tetraazacyclotetradecane, for example, are also useful, as are sparteine and hexamethylenetetraamine.

Although other acid salts are effective, it is preferred that the salt be a hydrohalide, especially a hydrochloride. Acid salts of tertiary amines which may be employed in the invention include, for example, diazabicyclo[2.2.2]octane dihydrochloride, 2,7-dimethyl-2,7-diazaoctane dihydrochloride, 2,3,4,6,7,8,9,10-octahydropyrimido[1,2-*a*]azepine hydrochloride, and quinuclidine hydrochloride.

The process of this invention is carried out between approximately equimolar amounts of the acyl halide, preferably the acyl chloride, aldehyde and aqueous solution of cyanide salt in the water-immiscible aprotic solvent, but slight excesses of the acyl halide and cyanide salt are typically used. The acyl halide may be added last, preferably dropwise, to the stirred reaction mixture, but it is preferred to add a solution containing aldehyde and acyl halide to a stirred mixture of aqueous cyanide salt and water-immiscible aprotic solvent. Although the reaction can be carried out over a wide temperature range, the range 0°C—50°C is satisfactory in most cases, and it is preferred to carry out the reaction at room temperature, since neither external heating nor cooling are then required.

The process will be understood more readily by reference to the following Examples, which illustrate it. Temperatures are in degrees Celsius. The reactions exemplified were, in many cases, monitored by gas liquid partition chromatography (glpc), and the time required for disappearance of the limiting reagent after beginning addition of the acyl halide was determined, together with the amount of alpha-cyano ester produced at that time.

Example 1

Preparation of α-cyano-3-phenoxybenzyl 3-(2,2-dichloroethenyl)-2,2-dimethylcyclopropanecarboxylate

*A. Using diazabicyclo[2.2.2]octane*

(1) A flask was charged with 3-phenoxybenzaldehyde (1.98 g, 10.0 mmole), sodium cyanide (0.59 g, 12 mmole), 20 ml n-heptane, 1 ml water, and diazabicyclo[2.2.2]octane (0.022 g, 0.2 mmole). This mixture was vigorously stirred, and 3-(2,2-dichloroethenyl)-2,2-dimethylcyclopropanecarbonyl chloride (2.38 g, 10.5 mmole) was added in one portion. Stirring was continued at room temperature for one hour, at which time glpc indicated a 94% yield of the desired alpha-cyano ester. The mixture was filtered. The filter cake was washed twice with 10 ml portions of diethyl ether. The filtrate was dried over magnesium sulfate, and the heptane was stripped under reduced pressure to yield the desired ester as a residual oil (4.10 g).

Use of the optically active (1R, *cis*) carbonyl chloride in the aforesaid process afforded the corresponding optically active ester in 96% yield within 1 hr. reaction time.

(2) Under a dry nitrogen atmosphere a mixture of sodium cyanide (5.9 g, 0.12 mole) and 1,4-diazabicyclo[2.2.2]octane (0.22 g, 0.002 mole) in 10 grams of water was stirred at room temperature. During a one hour period a solution of 3-phenoxybenzaldehyde (20.6 g, 0.1 mole) and 3-(2,2-dichloroethenyl)-2,2-dimethylcyclopropanecarbonyl chloride (24.1 g, 0.105 mole) in 120 ml of n-octane was added to the reaction mixture. After complete addition, the reaction mixture was stirred for one hour. An aqueous solution containing 20% sodium carbonate was added to the reaction mixture and the total warmed to 60°. The organic phase was separated and washed with water, dried over anhydrous magnesium sulfate and filtered. The solvent was removed from the filtrate under reduced pressure to yield α - cyano - 3 - phenoxybenzyl 3 - (2,2 - dichloroethenyl) - 2,2 - dimethylcyclopropanecarboxylate.

*B. Using 2,6-dimethyl-2,6-diazaheptane*

A flask was charged with a solution of 3-phenoxybenzaldehyde (1.98 g, 10 mmole) in 10 ml n-heptane. This solution was cooled to 10°, then 2,6-dimethyl-2,6-diazaheptane (26 mg, 0.2 mmole), sodium cyanide (0.59 g, 12 mmole), and 1 ml water were added. 2-(2,2-Dichloroethenyl)-2,2-dimethylcyclopropanecarbonyl chloride (2.38 g, 10.5 mmole) was then added in one portion with stirring, and the temperature of the reaction mixture was maintained at 8—12°. After 45 minutes, glpc indicated a 93% yield of the desired alpha-cyano ester. After a total of 1.25 hours, the reaction mixture was filtered. The filter cake was washed with ether, and the solvent was evaporated from the filtrate, affording the desired ester (4.3 g).

*C. Using 2,5,8,11-tetramethyl-2,5,8,11-tetraazadodecane*

A flask equipped with a stirrer, addition funnel, and an inlet for nitrogen gas was charged with sodium cyanide (0.59 g, 0.012 mole) dissolved in 1 ml water, 2,5,8,11-tetramethyl-2,5,8,11-tetraazadodecane (0.048 g, 0.2 mmole) in 3 ml heptane, and 3-phenoxybenzaldehyde (1.98 g, 0.01 mole) in 7 ml heptane. The reactants were mixed under a nitrogen atmosphere. 3-(2,2-Dichloroethenyl)-2,2-dimethylcyclopropanecarbonyl chloride (2.38 g, 0.0105 mole) in 10 ml heptane was added dropwise to the stirred mixture over a period of 20 minutes. Twenty minutes later, a total of 40 minutes, glpc indicated a 93% yield of the desired ester. The reaction mixture was stirred for another 40 minutes, then poured into a separatory funnel, diluted with 40 ml diethyl ether, washed once with a 1N aqueous solution of sodium hydroxide, three times with water, and once with saturated aqueous sodium chloride solution. After phase separation, the separated ethereal layer was dried over magnesium sulfate, and the solvent was evaporated, affording the ester as a pale yellow oil (4.02 g).

*D. Using 4,7,13,16,21-pentaoxa-1,10-diazabicyclo[8.8.5]tricosane*

A flask was charged with 3-phenoxybenzaldehyde (1.98 g, 10 mmole), 10 ml n-heptane, 4,7,13,16,21-pentaoxa-1,10-diazabicyclo[8.8.5]tricosane (66 mg, 0.2 mmole), sodium cyanide (0.59 g, 12 mmole), 1 ml water, and a solution of 3-(2,2-dichloroethenyl)-2,2-dimethylcyclopropanecarbonyl chloride (2.38 g, 10.5 mmole) in 10 ml n-heptane. The reaction mixture was stirred, and an exotherm (to approximately 40°) was observed after 20 minutes. After 50 minutes, glpc showed a 99% yield of the desired ester. The reaction mixture was filtered diluted with ether, dried over magnesium sulfate, and the solvent was evaporated to afford the desired ester (3.90 g).

*E. Using Tetramethylethylene Diamine*

A solution of 3-(2,2-dichloroethenyl)-2,2-dimethylcyclopropanecarbonyl chloride (114 g, 0.315 mole) and 3-phenoxybenzaldehyde (61.9 g, 0.3 mole) in 275 ml of n-heptane was added dropwise under nitrogen in one hour to a stirred mixture of sodium cyanide (18 g, 0.36 mole), tetramethylethylene diamine (0.7 g, 0.006 mole), and 30 g of water at about 40°. After the addition, the reaction mixture was stirred for 90 minutes at about 40°. The reaction mixture was worked up as in Example 1A.(2), producing the desired ester.

*F. Using Tetramethyl-1,6-hexane Diamine*

By the method of Example 1E. above, but substituting tetramethyl-1,6-hexane diamine for tetramethylethylene diamine, α-cyano-3-phenoxybenzyl 3-(2,2-dichloroethenyl)-2,2-dimethylcyclopropanecarboxylate was prepared.

The preparation of α-cyano-3-phenoxybenzyl 3-(2,2-dichloroethenyl)-2,2-dimethylcyclopropanecarboxylate using other tertiary amine or polyamine and cryptate rate-promoting agents under otherwise similar conditions gave the results shown in Table 1.

Example 2

Preparation of α-cyano-3-phenoxybenzyl 3-(2,2-dibromoethenyl)-2,2-dimethylcyclopropanecarboxylate using diazabicyclo[2.2.2]octane

A flask was charged with 3-phenoxybenzaldehyde (1.98 g, 10.0 mmole), 10 ml n-heptane, diazabicyclo-[2.2.2]octane (22 ml, 0.2 mmole), sodium cyanide (0.59 g, 12 mmole), and 1 ml water. 3-(2,2-Dibromoethenyl)-2,2-dimethylcyclopropanecarbonyl chloride (3.32 g, 10.5 mmole) in 10 ml n-heptane was added with stirring. After 2 hours, the reaction mixture was filtered, the filter cake was washed with 30 ml of ether, and the filtrate was dried over magnesium sulfate. The solvent was stripped, affording the desired ester as a yellow oil (4.4 g, 96% yield).

Example 3

Preparation of α-cyano-3-phenoxybenzyl 3-(2-chloro-3,3,3-trifluorophenyl)-2,2-dimethylcyclopropanecarboxylate using 2,7-dimethyl-2,7-diazaoctane

A flask was charged with 3-phenoxybenzaldehyde (1.98 g, 10 mmole), 10 ml n-heptane, 2,7-dimethyl-2,7-diazaoctane (29 mg, 0.2 mmole), sodium cyanide (0.59 g, 12 mmole) and 1 ml water. 3-(2-Chloro-3,3,3-trifluoropropenyl)-2,2-dimethylcyclopropanecarbonyl chloride (2.0 g, 10.5 mmole) dissolved in 10 ml n-heptane was then added in one portion. After 1.3 hr., the conversion was complete. The reaction mixture was stirred for 4.5 hr, when 5 ml diethyl ether and 5 ml 1N aqueous sodium hydroxide solution were added. The resultant mixture was stirred for 1.5 hr and the two phases separated. The organic phase was washed once with 1N aqueous sodium hydroxide solution, once with water, and once with a saturated aqueous sodium chloride solution, then dried over magnesium sulfate, filtered, and the solvent distilled off under reduced pressure to afford the desired ester (4.1 g, 91% yield).

Example 4

Preparation of α-cyano-3-phenoxybenzyl 2,2,3,3-tetramethylcyclopropanecarboxylate using 2,6-dimethyl-2,6-diazaheptane

A flask was charged with 3-phenoxybenzaldehyde (3.16 g, 16 mmole), 10 ml n-heptane, 2,6-dimethyl-2,6-diazaheptane (26 mg, 0.2 mmole), sodium cyanide (0.94 g, 19.2 mmole) and 1 ml water. 2,2,3,3-Tetramethylcyclopropanecarbonyl chloride (2.6 g, 16.7 mmole) dissolved in 10 ml n-heptane was added next, in one portion. An exotherm (to approximately 38°) was observed upon addition of the acid chloride. Analysis indicated a 74% yield of the desired ester in 4 hours. After cooling, the reaction mixture was filtered, diluted with diethyl ether, dried over magnesium sulfate, and distilled under reduced pressure to afford an orange residual oil. The oil was dissolved in 25 ml diethyl ether, and the resultant ethereal solution was mixed for 2 hr with a 1N aqueous sodium hydroxide solution. After phase separation, the ethereal layer was washed once with water, once with a saturated aqueous sodium chloride solution, dried over magnesium sulfate, and the solvent was distilled under reduced pressure to afford the desired ester (3.0 g).

Example 5

Preparation of α-cyano-3-phenoxybenzyl 2-(4-chlorophenyl)-3-methyl butanoate

*A. Using diazabicyclo[2.2.2]octane*

A flask was charged with 3-phenoxybenzaldehyde (1.98 g, 10 mmole), 10 ml n-heptane, diazabicyclo-[2.2.2]octane (22 mg, 0.2 mmole), sodium cyanide (0.59 g, 12 mmole), and 1 ml water, and stirring was begun. 2-(4-Chlorophenyl)-3-methylbutanoyl chloride (2.42 g, 10.5 mmole) in 10 ml n-heptane was then added in one portion. The reaction mixture was stirred at room temperature for 1 hr at which time glpc indicated a 74% yield of the desired ester. Stirring was continued overnight, then the reaction mixture was filtered, diluted with diethyl ether, and the solvent was distilled under reduced pressure to afford the desired ester as the residue (4.02 g).

*B. Using 2,5,8,11-tetramethyl-2,5,8,11-tetraazadodecane*

A flask was charged with 3-phenoxybenzaldehyde (1.98 g, 10 mmole), 20 ml n-heptane, 2,5,8,11-tetramethyl-2,5,8,11-tetraazadodecane (0.046 g), sodium cyanide (0.59 g, 12 mmole), and 1 ml. water. With stirring, 2-(4-chlorophenyl)-3-methylbutanoyl chloride (2.42 g, 10.5 mmole) was added dropwise over a period of 6 minutes. The reaction mixture was stirred at room temperature. Analysis by glpc 2.4 hr. after the acyl chloride had been added indicated a 67.7% yield of the desired ester. Stirring was continued overnight, and the desired ester (3.4 g) was isolated as described in the preceding Example.

Example 6

Preparation of α-cyano-3-phenoxybenzyl 3-(2,2-dichloroethenyl)-2,2-dimethylcyclopropanecarboxylate

*A. Using 1,4-piperazinebisethanesulfonic acid as the rate-promoting agent*

A flask was charged with 3-phenoxybenzaldehyde (1.98 g, 10 mmole), 10 ml n-heptane, 1,4-piperazinebisethanesulfonic acid (60 mg, 0.2 mmole), sodium cyanide (0.59 g, 12 mmole), 1 ml water, and a solution of 3-(2,2-dichloroethenyl)-2,2-dimethylcyclopropanecarbonyl chloride (2.38 g, 10.5 mmole) in 10 ml n-heptane. The reaction mixture was stirred for 4.5 hours (glpc indicated a 91% yield after 1 hr, 50 min), diluted with 30 ml diethyl ether, then washed once with 1N aqueous sodium hydroxide solution (20 ml), once with water (20 ml), and once with a saturated aqueous sodium chloride solution (20 ml). After separation, the clear yellow ethereal solution was dried over magnesium sulfate; then the solvent was evaporated to afford the desired ester (4.05 g).

*B. Using 3-(cyclohexylamino)propanesulfonic acid as the rate-promoting agent*

(1) In the manner of Example A above, but substituting 3-(cyclohexylamino)propanesulfonic acid (44 mg, 0.2 mmole) for the 1,4-piperazinebisethanesulfonic acid, glpc indicated a 97.2% yield of the desired ester after 95 minutes and isolation afforded the desired ester (3.49 g).

In a similar experiment, glpc indicated a 99% yield of the desired ester in two hours.

(2) A stirred mixture of sodium cyanide (18.0 g, 0.36 mole) and 3-(cyclohexylamino)propanesulfonic acid )1.34 g, 0.006 mole) in 300 ml of water was warmed to 40°. During a one hour period, maintaining a reaction temperature of about 40°, a solution of *cis*-3-(2,2-dichloroethenyl)-2,2-dimethylcyclopropanecarbonyl chloride (71.7 g, 0.315 mole) and 3-phenoxybenzaldehyde (61.9 g, 0.3 mole) in 262.2 g of n-heptane was added. The mixture was then stirred at 40° for one additional hour, after which it was washed with an aqueous solution containing 20% sodium carbonate and then with water. The organic phase was separated from the mixture and the solvent removed by distillation under reduced pressure to give α-cyano-3-phenoxybenzyl *cis*-3-(2,2-dichloroethenyl)-2,2-dimethylcyclopropanecarboxylate.

The substitution of other rate-promoting agents under otherwise similar conditions gave the following yields (glpc) after the indication reaction times.

| Rate-Promoting Agent | Reaction Time | Yield |
|---|---|---|
| 2-[4-(2-hydroxyethyl)piperazine-1-yl]ethanesulfonic acid | 55 min. | 91% |
| 4-pyridineethanesulfonic acid | 2.3 hr. | 94% |
| 2-[N,N-di-(2-hydroxy)ethyl]aminoethanesulfonic acid | 3.3 hr. | 90% |

## Example 7

Preparation of α-cyano-3-phenoxybenzyl 2-(4-chlorophenyl)-3-methyl butanoate using 3-(cyclohexylamino)propanesulfonic acid as the rate-promoting agent

A flask was charged with 3-(cyclohexylamino)propanesulfonic acid (42 mg), 3-phenoxybenzaldehyde (1.90 g, 9.6 mmole), sodium cyanide (0.56 g, 12 mmole), 1 ml water, and 15 ml n-heptane. 2-(4-Chlorophenyl)-3-methylbutanoyl chloride (2.34 g, 10.1 mmole) in 5 ml n-heptane was added dropwise over a period of 24 minutes to the stirred mixture. Thirty minutes after the addition was complete, a total of 54 minutes, glpc indicated a 95.6% yield of the desired ester. After stirring overnight, the reaction mixture was filtered and extracted with ether. The ether was evaporated from the extract, affording α-cyano-3-phenoxybenzyl 2-(4-chlorophenyl)-3-methyl butanoate (3.47 g).

## Example 8

Preparation of α-cyano-3-phenoxybenzyl 3-(2,2-dichloroethenyl)-2,2-dimethylcyclopropanecarboxylate using diazabicyclo[2.2.2]octane dihydrochloride as the rate-promoting agent

(1) A flask was charged with 3-phenoxybenzaldehyde (1.98 g, 10.0 mmole) 10 ml n-heptane, diaxabicyclo[2.2.2]octane dihydrochloride (30 mg, 0.2 mmole), sodium cyanide (0.59 g, 12 mmole), 1 ml water, and a solution of 3-(2,2-dichloroethenyl)-2,2-dimethylcyclopropanecarbonyl chloride (2.38 g, 10.5 mmole) in 10 ml n-heptane. The reaction mixture was stirred, and after 1.5 hours glpc indicated a 96% yield of the desired ester. After a total of 1 hr., 50 min, the reaction mixture was filtered, diluted with ether, the phases were separated, the ether phase wad dried over magnesium sulfate, and the solvent was evaporated to afford the desired ester (3.98 g).

(2) A stirred mixture of sodium cyanide (18.1 g, 0.36 mole) and diazabicyclo[2.2.2]octane dihydrochloride (1.11 g, 0.006 mole) in 30 g of water was warmed to 40°. During a one hour period a solution of 3-(2,2-dichloroethenyl)-2,2-dimethylcyclopropanecarbonyl chloride (77.6 g, 0.33 mole) and 3-phenoxybenzaldehyde (61.5 g, 0.3 mole) in 102 ml of n-heptane was added to the reaction mixture. After complete addition, the reaction mixture was stirred for 40 minutes and an additional 3.5 g (0.015 mole) of the acyl chloride added. The reaction mixture was stirred for an additional 40 minutes and then washed with 100 g of a 10% aqueous sodium carbonate solution and then water. The organic phase was separated from the mixture and the solvent removed by distillation under reduced pressure to give α-cyano-3-phenoxybenzyl 3-(2,2-dichloroethenyl)-2,2-dimethylcyclopropanecarboxylate.

The preparation of α-cyano-3-phenoxybenzyl 3-(2,2-dichloroethenyl)-2,2-dimethylcyclopropanecarboxylate using other rate-promoting agents under otherwise similar conditions gave the following results.

| Rate-Promoting Agent | Reaction Time | Yield |
|---|---|---|
| 2,7-dimethyl-2,7-diazaoctane dihydrochloride | 1.4 hr | 96% |
| 2,3,4,6,7,8,10-octahydropyrimido[1,2-*a*]azepine hydrochloride | 4 hr | 99% |
| quinuclidine hydrochloride | 1.8 hr | 98% |

TABLE 1

Preparation of α-cyano-3-phenoxybenzyl 3-(2,2-dichloroethenyl)-2,2-dimethylcyclopropanecarboxylate using other tertiary amine or polyamine and cryptate rate-promoting agents

| Rate-Promoting Agent | Reaction Time | Yield |
|---|---|---|
| tri-n-hexylamine | 4.5 hours | 82% |
| triethylamine | 2 hours | 89% |
| 2,4-dimethyl-2,4-diazapentane | 24 hours | 96% |
| 2,5-dimethyl-2,5-diazahexane | 0.5 hours | 91% |
| 2,9-dimethyl-2,9-diazadecane | 1.0 hour | 96% |
| 1,1′-(1,2-ethanediyl)bis[piperidine] | 1.0 hour | 94% |
| 1,4-dimethyl-1,4-diazacyclohexane | 1.0 hour | 86% |
| N,N,N′,N′-tetramethyl-1,2-diaminocyclohexane | 1.5 hours | 96% |
| 1,4,8,11-tetramethyl-1,4,8,11-tetraazacyclotetradecane | 1.5 hours | 96% |
| (−)-sparteine | 1.3 hours | 95% |

**Claims**

1. A method of preparing an alpha-cyano ester other than alpha - cyano - 3 - phenoxybenzyl 3 - (2,2 - dichloroethenyl) - 2,2 - dimethylcyclopropanecarboxylate, alpha - cyano - 3 - phenoxybenzyl 3 - (2,2 - dibromoethenyl) - 2,2 - dimethyl - cyclopropanecarboxylate, alpha - cyano - 3 - phenoxybenzyl 3 - (2 - chloro - 3,3,3 - trifluoropropenyl) - 2,2 - dimethylcyclopropanecarboxylate, alpha - cyano - 3 - phenoxybenzyl 2,2,3,3 - tetramethylcyclopropanecarboxylate, and alpha - cyano - 3 - phenoxybenzyl 2 - (4 - chlorophenyl) - 3 - methylbutanoate, comprising reacting an acyl halide with an aldehyde in a mixture of substantially water-immiscible aprotic solvent and an aqueous solution of water-soluble cyanide salt in the presence of a catalytic amount of rate-promoting agent that is a tertiary amine or polyamine, a cryptate, an amphoteric aminoalkylsulfonic acid surfactant, or acid salt of a tertiary amine.

2. A method according to Claim 1 in which an insecticidal alpha-cyano-3-phenoxybenzyl ester of the formula

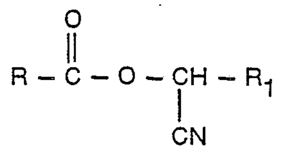

in which either (a) R is 3 - (2,2 - dichloroethenyl) - 2,2 - dimethylcyclopropyl, 3 - (2,2 - dibromoethenyl) - 2,2 - dimethylcyclopropyl, 3 - (2 - chloro - 3,3,3 - trifluoropropenyl) - 2,2 - dimethylcyclopropyl, 2,2,3,3 - tetramethylcyclopropyl, 1 - (4 - chlorophenyl) - 2 - methylpropyl, 1 - (4 - difluoromethoxy - phenyl) - 2 - methylpropyl, or [(2 - chloro - 4 - trifluoromethylphenyl) - amino] - 2 - methylpropyl and $R_1$ is 4 - fluoro - (3 - phenoxy)phenyl, 3 - (4 - halophenoxy)phenyl or 6 - phenoxy - 2 - pyridyl; or (b) R is 1 - (4 - difluoromethoxy - phenyl) - 2 - methylpropyl or 1 - [(2 - chloro - 4 - trifluoromethyl)amino] - 2 - methylpropyl and $R_1$ is 3-phenoxyphenyl, is prepared by reacting an acyl halide of the formula

$R—CO—X$

with an aldehyde of the formula $R_1—CH=O$, where R and $R_1$ are as defined above.

3. A process according to Claim 2, in which the rate-promoting agent is a linear tertiary polyamine of the formula

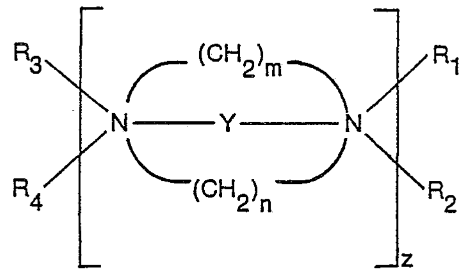

where Y is $—(CH_2)_k—$ with k being 1—6, $C_3—C_7$ cycloalkane, $C_2—C_6$ alkenyl, or $C_2—C_6$ alkynyl; and (a) z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; or (b) z is 2, m and n are 0, $R_1$ and $R_3$ are hydrocarbon groups and $R_2$ and $R_4$ are absent, 1,4,8,11-tetramethyl-1,4,8,11-tetraazacyclotetradecane, sparteine, or cryptates.

4. The process of claim 3 characterized in that the linear tertiary polyamine is 2,4-dimethyl-2,4-diazapentane, 2,5-dimethyl-2,5-diazahexane, 1,1′-(1,2-ethanediyl)-bis[piperidine], N,N,N′,N′-tetramethyl-1,2-diaminocyclohexane, 1,4-dimethyl-1,4-diazacyclohexane, diazabicyclo[2.2.2]octane, 2,6-dimethyl-2,6-diazaheptane, 2,7-dimethyl-2,7-diazaoctane, 2,9-dimethyl-2,9-diazadecane, or 2,5,8,11-tetramethyl-2,5,8,11-tetraazadodecane.

5. The process of claim 4 characterized in that the linear tertiary polyamine is diazabicyclo[2.2.2]octane, 2,6-dimethyl-2,6-diazaheptane, 2,7-dimethyl-2,7-diazaoctane, or 2,5,8,11-tetramethyl-2,5,8,11-tetraazadecane.

6. The process of claim 5 characterized in that the rate promoting agent is diazabicyclo[2.2.2]octane.

7. The process of claim 3 characterized in that the rate promoting agent is a cryptate of the formula

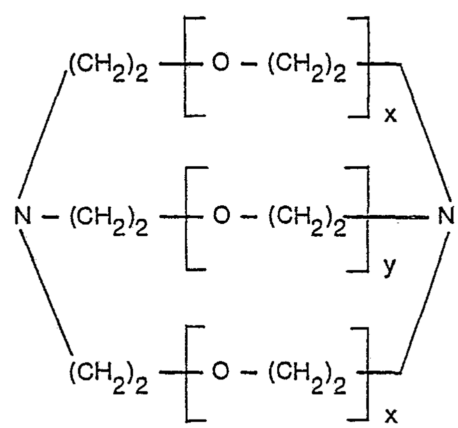

wherein x and y are independently 1 or 2.

8. The process of claim 7 characterized in that the cryptate is 4,7,13,16,21-pentaoxo-1,10-diazabicyclo-[8.8.5]tricosane.

9. A process according to any one of claims 3—8 characterized in that R is 3-(2,2-dichloroethenyl)-2,2-dimethylcyclopropyl.

10. A process according to any one of claims 3—8 characterized in that the water-immiscible aprotic solvent is n-heptane.

11. A process according to any one of claims 3—8 characterized in that the water-soluble cyanide salt is sodium cyanide.

12. The process of claim 2 characterized in that the rate-promoting agent is an aminoalkylsulfonic acid selected from 1,4-piperazinebisethanesulfonic acid, 4-pyridineethanesulfonic acid 2-[N,N-di-(2-hydroxy)-ethyl]aminoethanesulfonic acid 3-(cyclohexylamino)propanesulfonic acid, and 2-[4-(2-hydroxyethyl)-piperizine-1-yl]ethanesulfonic acid.

13. The process of claim 12 characterized in that the rate-promoting agent is selected from 1,4-piperizinebisethanesulfonic acid and 3-(cyclohexylamino)propanesulfonic acid.

14. The process of claim 13 characterized in that the rate-promoting agent is 3-(cyclohexylamino)-propanesulfonic agent.

15. A process according to any one of claims 12—14 characterized in that R is 3-(2,2-dichloroethenyl)-2,2-dimethylcyclopropyl or 1-(4-chlorophenyl)-2-methylpropyl.

16. A process according to any one of claims 12—14 characterized in that R is 3-(2,2-dichloroethenyl)-2,2-dimethylcyclopropyl.

17. A process according to any one of claims 12—14 characterized in that the water-immiscible aprotic solvent is n-heptane.

18. A process according to any one of claims 12—14 characterized in that the water-soluble cyanide salt is sodium cyanide.

19. The process of claim 2 characterized in that the rate-promoting agent is an acid salt of a tertiary amine selected from products of the reaction of a strong acid in a linear tertiary polyamine of the formula

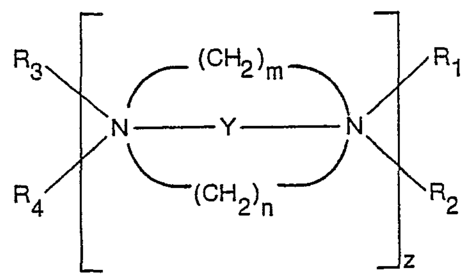

wherein Y is $—CH_2)_k—$ with k being 1—6, $C_3—C_7$ cycloalkane, $C_2—C_6$ alkenyl, or $C_2—C_6$ alkynyl; z is 1 or 2, and when z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R^4$ are absent.

20. The process of claim 19 characterized in that the rate-promoting agent is selected from diazabicyclo[2.2.2]octane dihydrochloride, 2,7-dimethyl-2,7-diazaoctane dihydrochloride, 2,3,4,6,7,8,9,10-octahydropyrimido[1,2-*a*]azepine hydrochloride, and quinuclidine hydrochloride.

21. The process of claim 20 characterized in that the rate-promoting agent is diazabicyclo[2.2.2]octane dihydrochloride.

22. A process according to any one of claims 19—21 characterized in that R is 3-(2,2-dichlorothenyl)-2,2-dimethylcyclopropyl.

23. A process according to any one of claims 19—21 characterized in that the water-immiscible aprotic solvent is n-heptane.

24. A process according to any one of claims 19—21 characterized in that the water-soluble cyanide salt is sodium cyanide.

25. A process according to any one of claims 3—24 characterized in that R is selected from 1-(4-difluoromethoxyphenyl)-2-methylpropyl and 1-[(2-chloro-4-trifluoromethyl)amino]-2-methylpropyl.

26. A process according to any one of claims 3—24 characterized in that the acyl halide is reacted with 4-fluoro-3-phenoxybenzaldehyde, a 3-(4-halophenoxy)benzaldehyde, or 6-phenoxy-2-pyridylcarboxaldehyde.

## Patentansprüche

1. Ein Verfahren zur Herstellung eines α - Cyanoesters, der nicht 3 - (2,2 - Dichloräthenyl) - 2,2 - dimethylcyclopropancarbonsäure - α - cyano - 3 - phenoxybenzylester, 3 - (2,2 - Dibromäthenyl) - 2,2 - dimethyl - cyclopropancarbonsäure - α - cyano - 3 - phenoxybenzylester, 3 - (2 - Chlor - 3,3,3 - trifluorpropenyl) - 2,2 - dimethylcyclopropancarbonsäure - α - cyano - 3 - phenoxybenzylester, 2,2,3,3 - Tetramethylcyclopropancarbonsäure - α - cyano - 3 - phenoxybenzylester und 2 - (4 - Chlorphenyl) - 3 - methylbutan-säure - α - cyano - 3 - phenoxybenzylester ist, gekennzeichnet durch die Umsetzung eines Säurehalogenids mit einem Aldehyd in einem Gemisch aus einem in wesentlichen mit Wasser nicht mischbaren aprotischen Lösungsmittel und einer wäßrigen Lösung eines wasserlöslichen Cyanidsalzes in Gegenwart einer katalytischen Menge eines die Umsetzungsrate fördernden Mittels, das ein tertiäres Amin oder Polyamin, ein Kryptat, ein amphoteres Aminoalkylsulfonsäure-Netzmittel oder ein Säuresalz eines tertiären Amins ist.

2. Verfahren nach Anspruch 1, bei dem ein insektizider α-Cyano-3-phenoxybenzylester der Formel

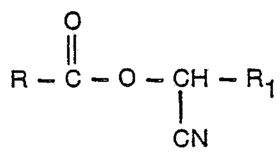

in dem entweder (a) R 3 - (2,2 - Dichloräthenyl) - 2,2 - dimethylcyclopropyl, 3 - (2,2 - Dibromäthenyl) - 2,2 - dimethylcyclopropyl, 3 - (2 - Chlor - 3,3,3 - trifluorpropenyl) - 2,2 - dimethylcyclopropyl, 2,2,3,3 - Tetramethylcyclopropyl, 1 - (4 - Chlorphenyl) - 2 - methylpropyl, 1 - (4 - Difluormethoxyphenyl) - 2 - methylpropyl oder 1 - [(2 - Chlor - 4 - trifluormethylphenyl) - amino] - 2 - methylpropyl bedeutet und $R_1$ 4 - Fluor - (3 - phenoxy) - phenyl, 3 - (4 - Halophenoxy) - phenyl oder 6 - Phenoxy - 2 - pyridyl darstellt, oder (b) R 1 - (4 - Difluormethoxyphenyl) - 2 - methylpropyl oder 1 - [(2 - Chlor - 4 - trifluormethyl) - amino] - 2 - methylpropyl bedeutet und $R_1$ 3-Phenoxyphenyl darstellt, durch Umsetzung eines Säurehalogenids der Formel

R—CO—X

mit einer Aldehyd der Formel $R_1$—CH=O hergestellt wird, wobei R und $R_1$ wie vorstehend definiert sind.

3. Verfahren nach Anspruch 2, wobei das die Umsetzungsrate fördernde Mittel ein lineares tertiäres Polyamin der Formel,

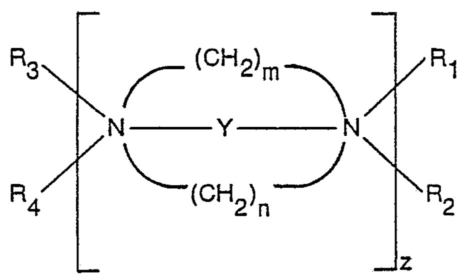

in der Y—$(CH_2)_k$— mit k im Wert von 1 bis 6, $C_3$—$C_7$-Cycloalkan, $C_2$—$C_6$-Alkenyl oder $C_2$—$C_6$-Alkinyl bedeutet und (a) z den Wert 1 hat, m und n 0 sind oder unabhängig voneinander einen Wert von 1 bis 6 haben und wenn m und n 0 sind, $R_1$, $R_2$, $R_3$ und $R_4$ Kohlenwasserstoffreste darstellen und $R_1$ mit $R_2$ und $R_3$ mit $R_4$ verbunden sein können, wobei ein Ring gebildet wird, der das Stickstoffatom enthält, an das sie beide gebunden sind, und wenn m einen Wert von 1 bis 6 hat und n 0 ist, die Reste $R_1$ und $R_3$ fehlen und $R_2$ und $R_4$ Kohlenwasserstoffreste bedeuten, und wenn sowohl m als auch n einen Wert von mindestens 1 haben, die Reste $R_2$ und $R_4$ fehlen, oder (b) z den Wert 2 hat, m und n 0 sind, $R_1$ und $R_3$ Kohlenwasserstoffreste bedeuten und $R_2$ und $R_4$ fehlen, oder 1,4,8,11-Tetramethyl-1,4,8,11-tetraazacyclotetradecan, Spartein oder ein Kryptat darstellt.

4. Verfahren nach Anspruch 3, gekennzeichnet dadurch, daß das lineare tertiäre Polyamin 2,4 - Dimethyl - 2,4 - diazapentan, 2,5 - Dimethyl - 2,5 - Dimethyl - 2,5 - dizahexan, 1,1' - (1,2 - Äthandiyl) - bis[piperidin], N,N,N',N' - Tetramethyl - 1,2 - diaminocyclohexan, 1,4 - Dimethyl - 1,4 - diazacyclohexan, Diazabicyclo[2.2.2]octan, 2,6 - Dimethyl - 2,6 - diazaheptan, 2,7 - Dimethyl - 2,7 - diazaoctan, 2,9 - Dimethyl - 2,9 - diazadecan oder 2,5,8,11 - Tetramethyl - 2,5,8,11 - tetraazadodecan ist.

5. Verfahren nach Anspruch 4, gekennzeichnet dadurch, daß das lineare tertiäre Polyamin Diazabicyclo[2.2.2] - octan, 2,6 - Dimethyl - 2,6 - diazaheptan, 2,7 - Dimethyl - 2,7 - diazaoctan, oder 2,5,8,11 - Tetramethyl - 2,5,8,11 - tetraazadecan ist.

6. Verfahren nach Anspruch 5, gekennzeichnet dadurch, daß das die Umsetzungsrate fördernde Mittel Diazabicyclo[2.2.2]octan ist.

7. Verfahren nach Anspruch 3, gekennzeichnet dadurch, daß das die Umsetzungsrate fördernde Mittel ein Kryptat der Formel

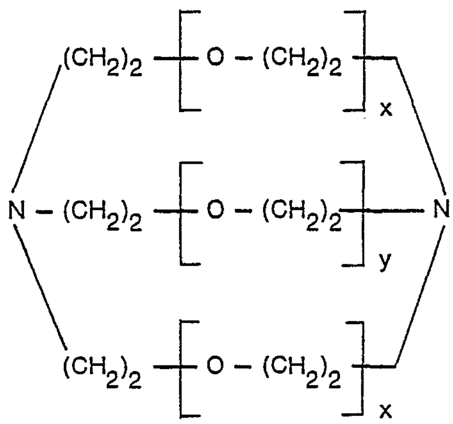

darstellt, in der x und y unabhängig voneinander den Wert 1 oder 2 haben.

8. Verfahren nach Anspruch 7, gekennzeichnet dadurch, daß das Kryptat 4,7,13,16,21-Pentaoxo-1,10-diazabicyclo[8.8.5] - tricosan ist.

9. Verfahren nach einem der Ansprüche 3 bis 8, gekennzeichnet dadurch, daß der Rest R 3-(2,2-Dichloräthenyl)-2,2-dimethylcyclopropyl ist.

10. Verfahren nach einem der Ansprüche 3 bis 8, gekennzeichnet dadurch, daß das mit Wasser nicht mischbare aprotische Lösungsmittel n-Heptan ist.

11. Verfahren nach einem der Ansprüche 3 bis 8, gekennzeichnet dadurch, daß das wasserlösliche Cyanidsalz Natriumcyanid ist.

12. Verfahren nach Anspruch 2, gekennzeichnet dadurch, daß das die Umsetzungsrate fördernde Mittel eine Aminoalkylsulfonsäure aus der Gruppe 1,4 - Piperazinbisäthansulfonsäure, 4 - Pyridinäthansulfonsäure, 2 - [N,N - di - (2 - Hydroxy) - äthyl] - aminoäthansulfonsäure, 3 - (Cyclohexylamino)propansulfonsaure und 2 - [4 - (2 - Hydroxyäthyl) - piperizin - 1 - yl] - äthansulfonsäure ist.

13. Verfahren nach Anspruch 12, gekennzeichnet dadurch, daß das die Umsetzungsrate fördernde Mittel 1,4-Plperizinbisäthansulfonsäure oder 3-(Cyclohexylamino)-propansulfonsäure ist.

14. Verfahren nach Anspruch 13, gekennzeichnet dadurch, daß das die Umsetzungsrate fördernde Mittel 3-(Cyclohexylamino)-propansulfonsäure ist.

15. Verfahren nach einem der Ansprüche 12 bis 14, gekennzeichnet dadurch, daß der Rest R 3 - (2,2 - Dichloräthenyl) - 2,2 - dimethylcyclopropyl oder 1 - (4 - Chlorphenyl) - 2 - methylpropyl ist.

16. Verfahren nach einem der Ansprüche 12 bis 14, gekennzeichnet dadurch, daß der Rest R 3-(2,2-Dichloräthenyl)-2,2-dimethylcyclopropyl ist.

17. Verfahren nach einem der Ansprüche 12 bis 14, gekennzeichnet dadurch, daß das mit Wasser nicht mischbare aprotische Lösungsmittel n-Heptan ist.

18. Verfahren nach einem der Ansprüche 12 bis 14, gekennzeichnet dadurch, daß das wasserlösliche Cyanidsalz Natriumcyanid ist.

19. Verfahren nach Anspruch 2, gekennzeichnet dadurch, daß das die Umsetzungsrate fördernde Mittel ein Säuresalz eines tertiären Amins ist, das ausgewählt ist aus den Reaktionsprodukten einer starken Säure und eines linearen tertiären Polyamins der Formel

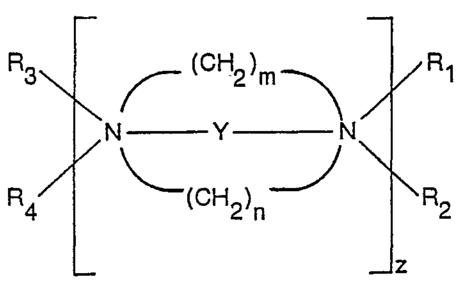

in der Y—$(CH_2)_k$— mit k im Wert von 1 bis 6, $C_3$—$C_7$-Cycloalkan, $C_2$—$C_6$-Alkenyl oder $C_2$—$C_6$-Alkinyl ist, z den Wert 1 oder 2 hat und wenn z den Wert 1 hat, m und n 0 sind oder unabhängig voneinander einen Wert von 1 bis 6 haben und wenn m und n 0 sind, $R_1$, $R_2$, $R_3$ und $R_4$ Kohlenwasserstoffreste sind und $R_1$ mit $R_2$ und $R_3$ mit $R_4$ verbunden sein können, wobei ein das Stickstoffatom, an das beide gebunden sind, enthaltenden Ring entsteht, und wenn m einen Wert von 1 bis 6 hat und n 0 ist, die Reste $R_1$ und $R_3$ fehlen und $R_2$ und $R_4$ Kohlenwasserstoffreste sind, und wenn sowohl m als auch n einen Wert von mindestens 1 haben, die Reste $R_2$ und $R_4$ fehlen, und wenn z den Wert 2 hat, m und n 0 sind, $R_1$ und $R_3$ Kohlenwasserstoffreste darstellen und $R_2$ und $R_4$ fehlen.

20. Verfahren nach Anspruch 19, gekennzeichnet dadurch, daß das die Umsetzungsrate fördernde

Mittel Diazabicyclo[2.2.2] - octan - dihydrochlorid, 2,7 - Dimethyl - 2,7 - diazaoctan - dihydrochlorid, 2,3,4,6,7,8,9,10 - Octahydropyrimido[1,2 - *a*]azepin - hydrochlorid oder Chinuclidin - hydrochlorid ist.

21. Verfahren nach Anspruch 20, gekennzeichnet dadurch, daß das die Umsetzungsrate fördernde Mittel Diazabicyclo[2.2.2]octan-dihydrochlorid ist.

22. Verfahren nach einem der Ansprüche 19 bis 21, gekennzeichnet dadurch, daß der Rest R 3-(2,2-Dichloräthenyl)-2,2-dimethylcyclopropyl ist.

23. Verfahren nach einem der Ansprüche 19 bis 21, gekennzeichnet dadurch, daß das mit Wasser nicht mischbare aprotische Lösungsmittel n-Heptan ist.

24. Verfahren nach einem der Ansprüche 19 bis 21, gekennzeichnet dadurch, daß das in Wasser lösliche Cyanidsalz Natriumcyanid ist.

25. Verfahren nach eine, der Ansprüche 3 bis 24, dadurch gekennzeichnet, daß R 1 - (4 - Difluor - methoxyphenyl) - 2 - methylpropyl oder 1 - [(2 - Chlor - 4 - trifluormethyl) - amino] - 2 - methylpropyl ist.

26. Verfahren nach einem der Ansprüche 3 bis 24, dadurch gekennzeichnet, daß das Acylhalogenid mit 4 - Fluor - 3 - phenoxybenzaldehyd, einem 3 - (4 - Halophenoxy)benzaldehyd oder 6 - Phenoxy - 2 - pyridylcarboxaldehyd umgesetzt wird.

**Revendications**

1. Procédé de préparation d'un alpha-cyanoester autre que le 3 - (2,2 - dichloréthényl) - 2,2 - diméthylcyclopropanecarboxylate d'alpha - cyano - 3 - phénoxybenzyle, le 3 - (2,2 - dibrométhényl) - 2,2 - diméthylcyclopropanecarboxylate d'alpha - cyano - 3 - phénoxybenzyle, le 3 - (2 - chloro - 3,3,3 - trifluoropropényl) - 2,2 - diméthylcyclopropanecarboxylate d'alpha - cyano - 3 - phénoxybenzyle, le 2,2,3,3 - tétraméthylcyclopropanecarboxylate d'alpha - cyano - 3 - phénoxybenzyle et le 2 - (4 - chlorophényl) - 3 - méthylbutanoate d'alpha - cyano - 3 - phénoxybenzyle, consistant à faire réagir un halogénure d'acyle avec un aldéhyde dans un mélange d'un solvant aprotique pratiquement non miscible à l'eau et d'une solution aqueuse d'un cyanure soluble dans l'eau en présence d'une quantité catalytique d'un agent favorisant la vitesse qui est une amine ou polyamine tertiaire, un cryptate, un agent tensio-actif amphotère d'acide aminoalkylsulfonique, ou un sel d'acide d'une amine tertiaire.

2. Procédé suivant la revendication 1, dans lequel un ester alpha-cyano-3-phénoxybenzylique répondant à la formule

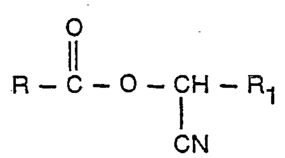

dans laquelle soit (a) R est un 3 - (2,2 - dichloréthényl) - 2,2 - diméthylcyclopropyle, un 3 - (2,2 - dibrométhényl) - 2,2 - diméthylcyclopropyle, un 3 - (2 - chloro - 3,3,3 - trifluoropropényl) - 2,2 - diméthylcyclopropyle, un 2,2,3,3 - tétraméthylcyclopropyle, un 1 - (4 - chlorophényl) - 2 - méthylpropyle, un 1 - (4 - difluorométhoxy - phényl) - 2 - méthylpropyle, ou un 1 - [(2 - chloro - 4 - trifluorométhylphényl)-amino] - 2 - méthylpropyle et $R_1$ est un 4 - fluoro - (3 - phénoxy)phényl, un 3 - (4 - halophénoxy)phényle ou un 6 - phénoxy - 2 - pyridyle ou (b) est un 1 - (4 - difluorométhoxy - phényl) - 2 - méthylpropyle ou un 1 - (2 - chloro - 4 - trifluorométhylpropyle et $R_1$ est un 3 - phénoxyphényle, est préparé en faisant réagir un halogénure d'acyle répondant à la formule

$$R—CO—X$$

avec un aldéhyde répondant à la formule $R_1—CH=O$, où R et $R_1$ sont tels que définis ci-dessus.

3. Procédé suivant la revendication 2, dans lequel l'agent favorisant la vitesse est une polyamine tertiaire linéaire répondant à la formule

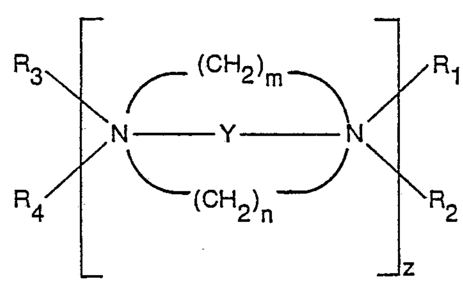

dans laquelle Y est $—(CH_2)_k$, k étant un 1—6, un cycloalkane en $C_3—C_7$, un alcényle en $C_2—C_6$ ou un alcynyle en $C_2—C_6$; et (a) z est 1, m et n sont 0 ou indépendamment 1—6, et lorsque m et n sont 0, $R_1$, $R_2$, $R_3$ et $R_4$ sont

des groupes hydrocarbonés, et $R_1$ peut être lié avec $R_2$, $R_3$ peut être lié avec $R_4$ pour former un cycle contenant l'atome N auquel tous deux sont liés, et lorsque m est 1—6 et N est 0, $R_1$ et $R_3$ sont absents, et $R_2$ et $R_4$ sont des groupes hydrocarbonés, et lorsque m et n sont tous deux au moins 1, $R_2$ et $R_4$ sont absents; ou (b) z est 2, m et n sont 0, $R_1$ et $R_3$ sont des groupes hydrocarbonés et $R_2$ et $R_4$ sont absents, un 1,4,7,11-tétraméthyl-1,4,8,11-tétraazacyclotétradécane, la spartéine ou des cryptates.

4. Procédé suivant la revendication 3, caractérisé en ce que la polyamine tertiaire linéaire est le 2 - 4 - diméthyl - 2,4 - diazapentane, le 2,5 - dyméthyl - 2,5 - diazahexane, la 1,1' - (1,2 - éthanediyl) - bis[pipéridine], le N,N,N',N' - tétraméthyl - 1,2 - diaminocyclohexane, le 1,4 - diméthyl - 1,4 - diazacyclohexane, le diazabicyclo[2.2.2]octane, le 2,6 - diméthyl - 2,6 - diazaheptane, le 2,7 - diméthyl - 2,7 - diazaoctane, le 2,9 - diméthyl - 2,9 - diazadécane, ou le 2,5,8,11 - tétraméthyl - 2,5,8,11 - tétraazadodécane.

5. Procédé suivant la revendication 4, caractérisé en ce que la polyamine tertiaire linéaire est le diazabicyclo[2.2.2]octane, le 2,6-diméthyl - 2,6 - diazaheptane, le 2,7 - diméthyl - 2,7 - diazaoctane, ou le 2,5,8,11 - tétraméthyl - 2,5,8,11 -tétraazadécane.

6. Procédé suivant la revendication 5, caractérisé en ce que l'agent favorisant la vitesse est le diazabicyclo[2.2.2]octane.

7. Procédé suivant la revendication 3, caractérisé en ce que l'agent favorisant la vitesse est un cryptate répondant à la formule:

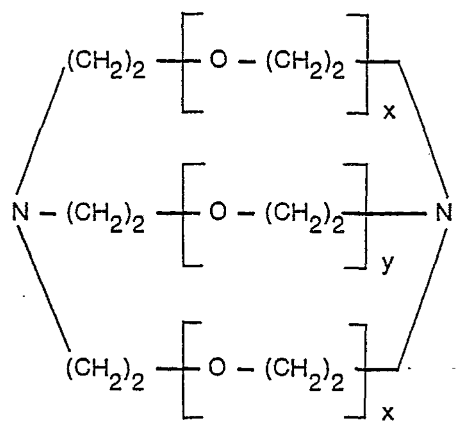

dans laquelle x et y sont indépendamment 1 ou 2.

8. Procédé suivant la revendication 7, caractérisé en ce que le cryptate est le 4,7,13,16,21-pentaoxo-1,10-diazabicyclo[8.8.5]tricosane.

9. Procédé suivant l'une quelconque des revendications 3—8 caractérisé en ce que R est un 3-(2,2-dichloréthényl)-2,2-diméthylcyclopropyle.

10. Procédé suivant l'une quelconque des revendications 3—8, caractérisé en ce que le solvant aprotique non miscible à l'eau est le n-heptane.

11. Procédé suivant l'une quelconque des revendications 3—8, caractérisé en ce que le cyanure soluble dans l'eau est le cyanure de sodium.

12. Procédé suivant la revendication 2, caractérisé en ce que l'agent favorisant la vitesse est un acide aminoalkylsulfonique choisi parmi l'acide 1,4-pipérazinebiséthanesulfonique, l'acide 4-pyridineéthanesulfonique, l'acide 2 - N,N - di - (2 - hydroxy) - éthylaminoéthanesulfonique, l'acide 3 - (cyclohexylamino)propanesulfonique, et l'acide 2 - 4 - (2 - hydroxyéthyl)pipérizine - 1 - yl éthanesulfonique.

13. Procédé suivant la revendication 12, caractérisé en ce que l'agent favorisant la vitesse est choisi parmi l'acide 1,4-pipérizinebiséthanesulfonique et l'acide 3 - (cyclohexylamino)propanesulfonique.

14. Procédé suivant la revendication 13, caractérisé en ce que l'agent favorisant la vitesse est l'acide 3-(cyclohexylamino)propanesulfonique.

15. Procédé suivant l'une quelconque des revendications 12—14, caractérisé en ce que R est un 3 - (2,2 - dichloroéthényl) - 22 - diméthylcyclopropyle ou un 1 - (4 - chlorophényl) - 2 - méthylpropyle.

16. Procédé suivant l'une quelconque des revendications 12—14, caractérisé en ce que R est un 3-(2,2-dichloréthényl)-2,2-diméthylcyclopropyle.

17. Procédé suivant l'une quelconque des revendications 12—14, caractérisé en ce que le solvant aprotique non miscible à l'eau est le n-heptane.

18. Procédé suivant l'une quelconque des revendications 12—14, caractérisé en ce que le cyanure soluble dans l'eau est le cyanure de sodium.

19. Procédé suivant la revendication 2, caractérisé en ce que l'agent favorisant la vitesse est un sel d'acide d'une amine tertiaire choisi parmi les produits de la réaction d'un acide fort et d'une polyamine tertiaire linéaire répondant à la formule

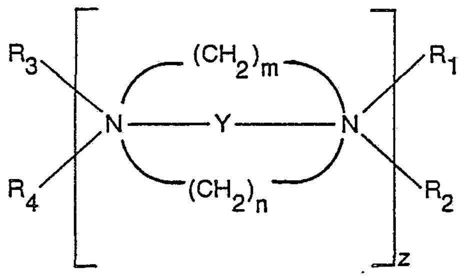

dans laquelle Y est $—(CH_2)_k$ avec k étant 1—6, un cycloalkane en $C_3—C_7$, un alcényle en $C_2—C_6$ ou un alcynyle en $C_2—C_6$; z est 1 ou 2, et lorsque z est 1, m et n sont 0 ou indépendamment 1—6, et lorsque m et n sont 0, $R_1$, $R_2$, $R_3$ et $R_4$ sont des groupes hydrocarbonés, et $R_1$ peut être lié $R_2$, et $R_3$ peut être lié avec $R_4$ pour former un cycle contenant l'atome N auquel tous deux sont liés, et lorsque m est 1—6 et n est 0, $R_1$ et $R_3$ sont absents, et $R_2$ et $R_4$ sont des groupes hydrocarbonés, et lorsque m et n sont tous deux au moins 1, $R_2$ et $R_4$ sont absents, et lorsque z est 2, m et n sont 0, $R_1$ et $R_3$ sont des groupes hydrocarbonés et $R_2$ et $R_4$ sont absents.

20. Procédé selon la revendication 19, caractérisé en ce que l'agent favorisant la vitesse est choisi parmi le dichlorhydrate de diazabicyclo[2.2.2]octane, le dichlorhydrate de 2,7-diméthyl-2,7-diazaoctane, le chlorhydrate de 2,3,4,6,7,8,9,10-octahydropyrimido[1,2-a]azépine, et le chlorhydrate de quinuclidine.

21. Procédé suivant la revendication 20, caractérisé en ce que l'agent favorisant la vitesse est le dichlorhydrate de diazabicyclo[2.2.2]octane.

22. Procédé suivant l'une quelconque des revendications 19—21, caractérisé en ce que R est un 3-(2,2-dichloréthényl)-2,2-diméthylcyclopropyle.

23. Procédé suivant l'une quelconque des revendications 19—21, caractérisé en ce que le solvant aprotique non miscible à l'eau est le n-heptane.

24. Procédé suivant l'une quelconque des revendications 19—21, caractérisé en ce que le cyanure soluble dans l'eau est le cyanure de sodium.

25. Procédé suivant l'une quelconque des revendications 3—24, caractérisé en ce que R est un 1 - (4 - difluorométhoxyphényl) - 2 - méthylpropyle ou un 1 - [(2 - chloro - 4 - trifluorométhyl)amino] - 2 - méthylpropyle.

26. Procédé suivant l'une quelconque des revendications 3—24, caractérisé en ce que l'halogénure d'acyle réagit sur le 4 - fluoro - 3 - phénoxybenzaldéhyde, sur un 3 - (4 - halophénoxy)benzaldéhyde ou sur le 6 - phénoxy - 2 - pyridylcarboxaldéhyde.